(19) Europäisches Patentamt
European Patent Office
Office européen des brevets

(11) **EP 1 230 669 B1**

(12) **EUROPEAN PATENT SPECIFICATION**

(45) Date of publication and mention
of the grant of the patent:
**06.01.2010 Bulletin 2010/01**

(21) Application number: **00935457.2**

(22) Date of filing: **07.06.2000**

(51) Int Cl.:
***H01J 61/28*** (2006.01)

(86) International application number:
**PCT/IL2000/000337**

(87) International publication number:
**WO 2000/075956 (14.12.2000 Gazette 2000/50)**

(54) **INFRA-RED LIGHT SOURCE**

INFRAROT-LICHTQUELLE

SOURCE DE LUMIERE INFRAROUGE

(84) Designated Contracting States:
**AT BE CH CY DE DK ES FI FR GB GR IE IT LI LU
MC NL PT SE**

(30) Priority: **08.06.1999 IL 13037299
04.04.2000 US 542768**

(43) Date of publication of application:
**14.08.2002 Bulletin 2002/33**

(73) Proprietor: **Oridion Breathid Ltd.
Jerusalem 91450 (IL)**

(72) Inventor: **COLMAN, Lewis
97430 Jerusalem (IL)**

(74) Representative: **Liesegang, Eva
Forrester & Boehmert
Pettenkoferstrasse 20-22
80336 München (DE)**

(56) References cited:
**EP-A- 0 415 600    GB-A- 1 591 709
US-A- 4 490 482    US-A- 4 639 432
US-A- 4 756 000    US-A- 4 757 512
US-A- 5 300 859**

**Description**

The present invention relates in general to the field of infra-red radiation sources, and in particular to sealed-off molecular gas discharge sources.

BACKGROUND OF THE INVENTION

The technology underlying the operation and production of electrode-less cold gas discharge infra-red lamp sources has been described in U.S. Patent No. 5,300,859, entitled "IR-Radiation Source and Method for Producing Same" to S. Yatsiv et al., hereby incorporated by reference in its entirety. One of the important advantages of such lamp sources is that they emit very narrow spectral lines at discrete frequencies characteristic of the molecular rotational-vibrational to ground state transitions of the excited gas species contained in the lamp. This is achieved in a source which is sealed-off, is compact, has a good level of conversion efficiency from electrical to optical power, and has a long life compared with previously available sealed-off lamps sources.

Because of their unique spectral properties, such lamps have been widely used as sources in non dispersive infra-red (NDIR) spectrometry instruments, and especially in gas analyzers for use in medical applications. The narrowness of the emission lines provides such gas analyzers with high levels of selectivity, sensitivity and stability, which are many times better than gas analyzers of similar complexity, which use alternative technology sources, such as hot blackbody sources. The other advantages mentioned above enable the production of compact and cost effective instrumentation using such sources.

The lamp sources described in U.S. Patent No. 5,300,859 have found particularly advantageous applications as sources of the $CO_2$ spectral emission lines, for gas analysis of exhaled breath, to determine the levels of $CO_2$ therein. Such $CO_2$ sources have been used to great advantage in capnography and breath testing instrumentation. Since the mechanisms and dynamics of the electrical excitation of $CO_2$ and of its ensuing spontaneous emission spectrum are similar to those used in carbon dioxide lasers, there exists a very large body of knowledge and prior art concerning this technology.

In U.S. Patent Nos. 4,757,512 and 4,756,000, both to J. Macken, there is respectively described the use of a silver oxide and a gold catalyst to oxidize carbon monoxide to form $CO_2$, this having particular application to $CO_2$ lasers.

In GB Patent No. 1,591,709, to R.S. Webley, there is described a carbon monoxide and/or carbon dioxide gas discharge tube containing a heated carbon filament and/or a heated filament comprising an oxygen generator such as potassium chlorate, to selectively or continuously provide carbon and/or oxygen for a regeneration cycle to compensate for dissociation of the carbon monoxide and/or carbon dioxide of the discharge tube into their constituent elements.

In European Patent No. EP 0,415,600, to R.C. Sharp, there is described a carbon dioxide waveguide laser, incorporating a Deuterium catalyst in the lasing mixture to catalyze the recombination of CO and $O_2$ back to $CO_2$, thereby increasing power output from the laser.

In U.S. Patent No. 4,490,482 to P. Mathieu, there is described a catalyst for recombining oxygen and carbon monoxide in a sealed $CO_2$ laser, and including a stannic oxide base and a coating of at least one metal selected from a group consisting of platinum, palladium, ruthenium, gold and rhodium.

In U.S. Patent No. 4,639,432 to A. Holt et al., there are described catalysts adapted to catalyze the oxidation of carbon monoxide to carbon dioxide, such as may be used to effect the combination of carbon monoxide and oxygen in carbon dioxide gas lasers.

In U.S. Patent No. 5,300,859 to S. Yatsiv et al. , there is a thorough discussion regarding the parameters affecting the lamp emission rise and decay time, efficiency, excitation and output, and the lamp lifetime as a function of chemical methods used to clean the lamp before sealing. On the other hand, the question of the spectral stability of the lamp source is not addressed. However, when used as a frequency selective source in NDIR spectroscopic applications, spectral stability may be even more important than the above parameters. Intensity changes over time can easily be monitored and corrected by using a reference path, since lamp intensity is a single valued quantity. On the other hand, spectral changes can not be easily monitored or corrected for, because there is virtually infinite information in a spectrum. Changes in the lamp spectrum cause changes in the absorption cell absorption characteristics. If these changes are not known, then it is impossible to accurately measure gas concentrations using such lamp sources.

There therefore exists a serious need for a method of maintaining a high level of spectral stability in electrode-less cold gas discharge infra-red lamp sources of the type described in U.S. Patent No. 5,300,859.

The disclosures of all publications mentioned in this section and in the other sections of the specification, are hereby incorporated by reference, each in its entirety.

SUMMARY OF THE INVENTION

The present invention seeks to provide a new method of producing cold gas discharge infra-red lamp sources with improved spectral stability, especially those operating with a carbon dioxide fill.

The spectral stability of the lamp source is of particular importance for use in such applications as in the gas analyzer of breath test instrumentation, where high resolution, sensitivity and selectivity are required to accurately determine the concentration of one isotopic species in the presence of another. Lack of good spectral stability may cause small changes occurring in the source spectrum to be erroneously interpreted as intensity changes resulting from changes in isotopic concentration. It is to be understood though, that such lamps may be used in any gas analyzer application to provide high resolution, sensitivity and selectivity.

A number of factors affect the spectral stability of the electrode-less cold gas discharge infra-red lamp source, of the type used in the breath test instrumentation according to the present invention. These factors include temperature, electrode position and time. The first two are factors of the operating conditions and geometry of the lamp, and if well understood, can be well controlled. The third factor mentioned, namely change over time, is much more problematic, since such changes are due to long term changes in the composition of the gas fill. It is thought that a major cause for long term changes in the lamp output and spectrum is the result of the gradual break down of the IR-active molecule in the discharge. In the case of the carbon dioxide lamp, the carbon dioxide dissociates into carbon monoxide and oxygen. In the course of the first few minutes of lamp operation, an equilibrium of the above molecules is reached, but over a longer period of operation, this equilibrium level changes as CO and $O_2$ are adsorbed on the walls of the lamp envelope. Impurities also reduce the $CO_2$ level still further.

Up to now, the majority of the prior art on the subject of the changes with time in the operating conditions of electric discharges in carbon dioxide has been primarily concerned with the change in power output which occurs as the $CO_2$ level changes. This has been the main point of interest because of the importance of avoiding a decay over time in the power levels of $CO_2$ lasers, and especially of sealed-off $CO_2$ lasers.

However, in the case of discharge lamps, whose emitted radiation is a result of non-coherent, spontaneous emission from an excited state to the ground state, changes in $CO_2$ concentration also affect the emission spectrum by means of a process known as self-absorption. In $CO_2$ lasers, on the other hand, this phenomenon is, for all practical purposes, virtually non-existent. The phenomenon of self-absorption operates in the following way. The $CO_2$ molecules in the lamp not only emit radiation when excited, but they also absorb radiation when in the ground state, by means of induced absorption. The lamp itself thus operates as an absorption cell to its own emitted light as this light passes through the lamp's own gas fill to the output window. The $CO_2$ lines are absorbed at their centers, and their shapes thus change by means of the process known as self-absorption.

The effect of self absorption on NDIR spectroscopy measurements can be significant. Even when Doppler broadened, the lines emitted from the lamp are much narrower than the absorbing lines in a gas sample at atmospheric pressure. As a result, the region of coincidence on the absorbing lines is of approximately constant magnitude. Hence, although there is no change in absorption characteristics for any individual emission line for different degrees of self-absorption, the change in the distribution of the individual line intensities does cause an overall change in the absorption characteristics. As a result, changes in self-absorption create a change in the emitted line strength distribution of the first order band group, i.e. the weaker absorbing lines of the Boltzman distributed intensities traverse the lamp with little attenuation, even in the presence of the high $CO_2$ concentrations inside the lamp, but the strongly absorbing lines of the Boltzman distributed intensities are strongly attenuated.

These changes in line intensity distribution are similar in their effect on the absorption characteristics to changes in distribution resulting from changes in relative group/order (isotope) strength, or Boltzman distribution changes resulting from changes in temperature.

Expressed mathematically, the lamp output radiation after absorption in the gas cell is given by:

$$I = \Sigma e^{-\alpha 1jdc} I_{1j} + \Sigma e^{-\alpha 2jdc} I_{2j}$$

where $I_1$ and $I_2$ are for the first and second order respectively, and are defined over their line intensities $I_{nj}$. This relationship follows from the well-known Beer-Lambert law.

Defining a distribution ratio $X = \Sigma I_{1j} /(\Sigma I_{1j} + \Sigma I_{2j})$, which is the output ratio between first and second order of line distribution j, the transmission $t_{(c)}$ is given by:

$$t_{(c)} = t_{1(c)} X + t_{2(c)} (1-X)$$

for any given concentration c.

When the lamp $CO_2$ concentration changes with time, this distribution ratio changes and hence, also the absorption characteristics of the complete optical system, consisting of the emitting lamp together with the absorbing cell. Under these conditions, it becomes difficult to distinguish such changes from the changes in gas concentration being measured.

Although self-absorption can be anticipated from theory, it is difficult to demonstrate directly. This is mainly because the typical line width of the lamp is less than $0.006 cm^{-1}$. The only instrument currently available which is capable of resolving such a narrow width is an FTIR (Fourier transform infra-red) spectrometer, with a mirror movement of at least 2 meters. The self absorption fine structure is even more difficult to resolve.

Experiments have been performed to observe the effect in discharge lamps, by using an FTIR spectrometer with possibly one of the optimum resolutions currently attainable. Comparisons between aged and new lamps for changes in line shape clearly show the effect. Other effects such as changes in Boltzman distribution, or changes in the ratio of first and second order lines, are shown to be negligible in comparison.

It is well-known in the art that one method of encouraging the recombination of dissociation products of molecules broken down under the effect of electrical discharges, to reproduce the parent gas molecule from which they originally dissociated, is by the use of catalysts. In the case of the $CO_2$ discharge, the carbon monoxide and oxygen molecules within the lamp envelope can be recombined under the influence of suitable catalysts, to reform the $CO_2$ molecules from which they dissociated. In sealed-off laser $CO_2$ laser technology, such catalysts are widely used to maintain the level of $CO_2$ in the laser cavity, in order to prevent a decay in the laser power output which would occur if the percentage of dissociated $CO_2$ were to increase with time.

As mentioned above, the effect of self-absorption is effectively non-existent in $CO_2$ laser discharges, as also in other systems not lasing directly to the ground state. In the $CO_2$ laser, the stimulated emission of the laser light is produced by a decay transition from a metastable state down to a short-lived excited state. It is this transition which produces the familiar $CO_2$ 10.6$\mu$m wavelength radiation. Since the lasing transition is not to the ground state, the large population of ground state molecules do not absorb the lasing transition energy, and for this reason, self-absorption is effectively non-existent in such laser discharges.

In gas discharge lamps of the type disclosed in U.S. Patent No. 5,300,839, on the other hand, the IR radiation is created by spontaneous emission in the wavelength region of 4.3$\mu$m, by transition of the IR active molecules from rotational-vibrational excited states, directly to the ground state. No metastable states are involved in this transition scheme. Since a high proportion of the $CO_2$ molecules populate the ground level, the spontaneous radiation associated with the transition to this ground level is readily absorbed by these ground state molecules, by the process of induced absorption. This results in appreciable self-absorption of the radiation. In this respect, discharge lamps are significantly different from the stimulated emission of laser discharges operating in the same molecular system, in that they show a significant self absorption effect.

The previously known catalyst technology used in laser discharges is aimed exclusively at maintaining the laser gain, the laser efficiency and the power output level of the emission from the laser. To the best of the Applicants' knowledge, no mention has been made or suggested, that such catalysts be used to maintain the spectral stability of the discharge, since the mechanism of self-absorption by which this could be performed, is not applicable to lasers. Furthermore, to the best of the Applicants' knowledge, catalysts have never been used, or their use suggested for stabilizing the emission spectra of gas discharge lamps.

According to the present invention, the aforementioned problems are solved with a method of constructing a lamp and a lamp according to claims 1 and 10, respectively.

There is thus provided, in accordance with a preferred embodiment of the present invention, a method for increasing the spectral stability of cold gas discharge infra-red lamp sources, by the use of a catalyst to reduce the changes with time in the concentration of excited gas molecules in the lamp active in emitting to ground state levels, thereby resulting in a reduction in the changes in self-absorption.

In accordance with a further preferred embodiment of the method of the present invention, there is provided an additional step of including a catalytic material within the lamp envelope, such that the volume of the lamp can be decreased.

In accordance with another preferred embodiment of the method of the present invention, there is provided the additional step of including a catalytic material within the lamp envelope, such that the IR-active gas concentration can be decreased.

There is further provided in accordance with yet another preferred embodiment of the present invention, any of the methods described above, wherein the catalytic material is operative to increase the spectral stability of the lamp by reducing changes with time in the level of self-absorption in the gas mixture.

Furthermore, in accordance with a preferred embodiment of a lamp of the present invention, there is provided a lamp wherein the catalyst is operative to increase the spectral stability of the lamp by reducing changes with time in the level of self-absorption in the gas mixture.

Furthermore, in accordance with another preferred embodiment of the lamp of the present invention, there is provided a lamp, wherein the IR-active gas species is carbon dioxide. The concentration of the carbon dioxide may be less than approximately 5%.

In accordance with still more preferred embodiments of the present invention, the catalytic material may be coated on an inside wall of the envelope, and may be gold, silver, rhodium, iridium, palladium, platinum or nickel.

There is provided in accordance with yet a further preferred embodiment of the present invention, any of the methods described above, wherein the lamp volume is less than approximately 6 milliliters.

BRIEF DESCRIPTION OF THE DRAWINGS

The present invention will be understood and appreciated more fully from the following detailed description, taken in conjunction with the drawing in which:

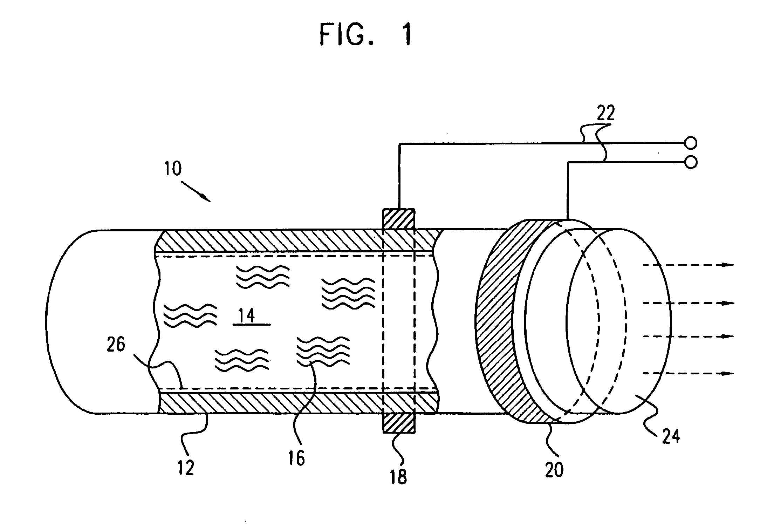

Fig. 1 is a schematic drawing of an improved electrically-excited gas discharge lamp, constructed and operative according to a preferred embodiment of the present invention.

## DETAILED DESCRIPTIONS OF PREFERRED EMBODIMENTS

A number of catalysts are known for reproducing $CO_2$ from CO and $O_2$, such as platinum with tin oxide, sputtered gold and silver coatings. The method of providing the catalytic coating on the interior of the discharge lamp envelope, in order to increase the lamp's spectral stability, depends on the material used for the catalyst. According to one preferred embodiment of the present invention, the method consists of sputtering the gold in a finely divided form onto the inside of the lamp envelope, such that it forms a non-conducting film, with a very high surface to volume ratio. Other metals such as Iridium, Rhodium, Palladium and Nickel can also be used as catalysts. Other methods of applying the catalyst besides sputtering may also be used, such as chemical or vapor deposition.

Reference is now made to Fig. 1, which is a schematic drawing of an improved electrically-excited cold-cathode gas discharge lamp 10, constructed and operative according to another preferred embodiment of the present invention. The basic elements of the lamp construction are similar to those disclosed in U.S. Patent No. 5,300,859. The envelope 12 of the lamp defines an internal volume 14 which contains a gaseous mixture 16 including one or more IR-active gaseous species. The gas is preferably excited by two electrodes 18, 20, disposed outside of the envelope, and supplied with RF-exciting power via a pair of cables 22. After being raised to an excited state, the IR-active gaseous species decays by means of a spontaneous emission directly to the ground state, and the emitted radiation is output by means of an optically transparent window 24 at one end of the lamp envelope. According to this preferred embodiment of the present invention, on the inner wall of the lamp envelope is a layer of catalyst 26, operative to maintain a constant level of the dissociation products of the IR-active gaseous species, such that the self absorption of the emitted radiation is kept at a constant level, and spectral stability of the lamp output maintained, as explained in detail hereinabove.

The catalyst is chosen to ensure an equilibrium between the IR-active gaseous species and its dissociation products. In this respect, it should be noted that unlike prior art use of catalysts in lasers, where the catalyst is optimally operative to keep dissociation of the IR-active species to a minimum, in order to keep laser output power to a maximum, according to the present invention, the catalyst need only maintain a constant equilibrium level of the IR-active species in order to achieve its aim of maintaining constant self-absorption and hence constant spectral stability. The catalyst may be applied by any of the methods known in the art, by chemical, sputtered or vapor deposition, or by any other suitable means. The catalyst may be of any of the types mentioned hereinabove.

Since the catalyst reduces the breakdown of $CO_2$, it is possible to reduce the need for a large ballast volume of gas, and thus to produce a lamp of considerably reduced size. Such a lamp is advantageous for use in portable systems. Such a smaller lamp has a better surface to volume ratio with respect to the active media and the activated $O_2$ molecules require a shorter path length to reach the coating, and hence have a higher probability of reaching the catalytic coating in the required activated state. In U.S. Patent No. 5,300,859, lamp volumes of the order of 60 ml. are disclosed. This volume was required to ensure an adequate reservoir volume to ensure long lamp life. If lamps of smaller volume were used, the increased effect of the absorption of dissociated carbon monoxide and oxygen on the increased surface of the walls in relation to the gas volume, would result in the lamp discharge decaying very rapidly. The use of a catalyst on the lamp walls, according to a further embodiment of the present invention, allows significantly smaller lamps to be constructed, without negatively affecting their lifetime or spectral stability. It is possible to achieve lamp volumes of 6ml or less, which provide similar lifetimes to those of the prior art 60ml volume lamps, while maintaining the same level of spectral stability. This is of significant advantage for use in the breath tester, where two lamp sources are typically required, whose mutual stability is dependent on the maintenance of both of them under similar environmental conditions, something that is simpler to achieve with more compact lamps.

Since the catalyst reduces the breakdown of $CO_2$, the use of a catalyst according to the present invention, makes it feasible to produce a lamp with a lower $CO_2$ pressure. In U.S. Patent No. 5,300,859, $CO_2$ percentages of the order of 10% are recommended for optimum output and lifetime considerations. According to preferred embodiments of the present invention, the use of a catalyst allows the operation of the lamp with lower $CO_2$ concentrations in the lamp gas mixture, down to 5%. This results in lower self-absorption effects, with consequent higher intensity central lines of the absorption spectrum, as explained hereinabove. This provides very deep absorption curves with a high extinction ratio in the gas analyzer measurement cell, permitting use of a shorter cell path and a more compact instrument without losing detection sensitivity or selectivity. This is of high importance when measuring the very low concentration levels of the minority isotopic component in a breath test.

It will be appreciated by persons skilled in the art that the present invention is not limited by what has been particularly shown and described hereinabove. Rather the scope of the present invention is defined by the appended claims.

## Claims

1. A method of constructing an electrically excited, gas discharge lamp, comprising the steps of:

constructing a lamp envelope (12);
cleaning said lamp envelope; and
filling said envelope with a gas mixture (16) comprising at least one IR-active gas species, said gas species being such that said lamp provides an output characteristic of spontaneous emission to a ground state when electrically excited;

said method being **characterized by** the additional step of including a catalytic material (26) within said lamp envelope so as to reduce changes with time in the concentration of said IR-active gas species.

2. The method according to claim 1, wherein said catalytic material (26) is operative to increase the spectral stability of said lamp by reducing changes with time in the level of self-absorption in said gas mixture.

3. The method according to claim 1 wherein said catalytic material (26) is coated on an inside wall of said envelope (12).

4. The method according to claim 1, wherein said catalytic material (26) is chosen from a group comprising gold, silver, rhodium, iridium, palladium, platinum and nickel.

5. A method according to claim 1 and wherein said step of including a catalytic material (26) within said lamp envelope (12) enables the volume of said lamp to be reduced compared to a lamp constructed without including such a catalytic material.

6. The method according to claim 5, wherein said lamp volume is less than approximately 6 milliliters.

7. A method according to claim 1 and wherein said step of including a catalytic material (26) within said lamp envelope enables the IR-active gas concentration to be reduced compared to a lamp constructed without including such a catalytic material (26).

8. The method according to any of the previous claims wherein said IR-active gas species is carbon dioxide.

9. The method according to claim 8, wherein the concentration of said carbon dioxide is less than approximately 5%.

10. An electrically excited gas discharge lamp, comprising:

a lamp envelope (12) containing a gas mixture (16) comprising said at least one IR-active gas species, said gas species being such that said lamp provides an output characteristic of spontaneous emission to a ground state when electrically excited; and

electrodes (18, 20) external to said envelope (12) for exciting said at least one IR-active gas species;

said lamp being **characterized by** a catalytic material (26) located within said lamp envelope (12) so as to reduce changes with time in the concentration of said IR-active gas species.

11. The lamp according to claim 10, wherein said catalyst (26) is operative to increase the spectral stability of said lamp by reducing changes with time in the level of self-absorption in said gas mixture.

12. The lamp according to claim 10, wherein said catalytic material (26) is coated on an inside wall of said envelope (12).

13. The lamp according to claim 10, wherein said catalytic material (26) is chosen from a group comprising gold, silver, rhodium, iridium, palladium, platinum and nickel.

14. The lamp according to any of claims 10 to 13, wherein said IR-active gas species is carbon dioxide.

15. The method according to claim 8 as applicable to claim 7 and wherein said reduced concentration of carbon dioxide reduces the self-absorption of said lamp, such that a gas analyzer utilizing the carbon dioxide emission spectrum of said lamp shows increased absorption curve depths compared to an analyzer utilizing a lamp constructed without including such a catalytic material (26).

**Patentansprüche**

1. Verfahren zur Konstruktion einer elektrisch angeregten Gasentladungslampe, umfassend die Schritte:

Konstruktion eines Lampenmantels (12);
Reinigung des Lampenmantels; und
Füllung des Mantels mit einem Gasgemisch (16), das mindestens eine IR-aktive Gasart umfasst, wobei die Gasart so ist, dass die Lampe eine für eine Spontanemission charakteristische Ausgabe an einen Grundzustand bereitstellt, wenn sie elektrisch angeregt wird.
wobei das Verfahren durch den zusätzlichen Schritt **gekennzeichnet** ist, ein Katalysatormaterial (26) innerhalb des Lampenmantels einzulagern, um mit der Zeit auftretende Veränderungen der Konzentration der IR-aktiven Gasart zu reduzieren.

2. Verfahren nach Anspruch 1, wobei das Katalysatormaterial (26) wirksam ist, die Spektralstabilität der Lampe zu erhöhen, indem mit der Zeit auftretende Veränderungen des Grades der Eigenabsorption bei dem Gasgemisch reduziert werden.

**3.** Verfahren nach Anspruch 1, wobei das Katalysatormaterial (26) auf eine Innenwand des Lampenmantels (12) aufgetragen wird.

**4.** Verfahren nach Anspruch 1, wobei das Katalysatormaterial (26) ausgewählt ist aus einer Gruppe umfassend Gold, Silber, Rhodium, Iridium, Palladium, Platin und Nikkel.

**5.** Verfahren nach Anspruch 1 und wobei der Schritt, ein Katalysatormaterial (26) innerhalb des Lampenmantels (12) einzulagern, eine Reduzierung des Volumens der Lampe ermöglicht, verglichen mit einer Lampe, die ohne Einlagerung eines solchen Katalysatormaterials konstruiert ist.

**6.** Verfahren nach Anspruch 5, wobei das Lampenvolumen kleiner als etwa 6 Milliliter ist.

**7.** Verfahren nach Anspruch 1 und wobei der Schritt, ein Katalysatormaterial (26) innerhalb des Lampenmantels einzulagern, eine Reduzierung der Konzentration des IR-aktiven Gases ermöglicht, verglichen mit einer Lampe, die ohne Einlagerung eines solchen Katalysatormaterials (26) konstruiert ist.

**8.** Verfahren nach einem der vorhergehenden Ansprüche, wobei die IR-aktive Gasart Kohlendioxid ist.

**9.** Verfahren nach Anspruch 8, wobei die Kohlendioxidkonzentration kleiner als etwa 5 % ist.

**10.** Elektrisch angeregte Gasentladungslampe, umfassend:

einen Lampenmantel (12) mit einem Gasgemisch (16), das mindestens eine IR-aktive Gasart umfasst, wobei die Gasart so ist, dass die Lampe eine für eine Spontanemmission charakteristische Ausgabe an einen Grundzustand bereitstellt, wenn sie elektrisch angeregt wird. Elektroden (18, 20) außerhalb des Mantels (12) zum Anregen der mindestens einen IR-aktiven Gasart;
wobei die Lampe **dadurch gekennzeichnet ist, dass** ein Katalysatormaterial (26) innerhalb des Lampenmantels (12) angeordnet ist, um mit der Zeit auftretende Veränderungen der Konzentration der IR-aktiven Gasart zu reduzieren.

**11.** Lampe nach Anspruch 10, wobei der Katalysator (26) wirksam ist, die Spektralstabilität der Lampe zu erhöhen, indem mit der Zeit auftretende Veränderungen des Grades der Eigenabsorption bei dem Gasgemisch reduziert werden.

**12.** Lampe nach Anspruch 10, wobei das Katalysatormaterial (26) auf eine Innenwand des Lampenmantels (12) aufgetragen wird.

**13.** Lampe nach Anspruch 10, wobei das Katalysatormaterial (26) ausgewählt ist aus einer Gruppe umfassend Gold, Silber, Rhodium, Iridium, Palladium, Platin und Nikkel.

**14.** Lampe nach einem der vorhergehenden Ansprüche 10 bis 13, wobei die IR-aktive Gasart Kohlendioxid ist.

**15.** Verfahren nach Anspruch 8, wie auf Anspruch 7 anwendbar und wobei die reduzierte Konzentration von Kohlendioxid die Eigenabsorption der Lampe so reduziert, dass ein Gasanalysator, der das Emissionsspektrum an Kohlendioxid der Lampe nutzt, eine erhöhte Absorptionskurventiefe zeigt verglichen mit einem Analysator, der eine Lampe nutzt, die ohne Einlagerung eines solchen Katalysatormaterials (26) konstruiert ist.

**Revendications**

**1.** Méthode de construction d'une lampe à décharge de gaz excitée électriquement, comprenant les étapes suivantes :

construction d'une enveloppe de lampe (12) ; nettoyage de ladite enveloppe de lampe ; et remplissage de ladite enveloppe avec un mélange de gaz (16) comprenant au moins une espèce de gaz à IR activé, ladite espèce de gaz étant telle que ladite lampe fournit une caractéristique de sortie d'émission spontanée à un état de base lorsqu'elle est électriquement excitée ; ladite méthode étant **caractérisée par** l'étape additionnelle consistant à inclure un matériau catalytique (26) au sein de ladite enveloppe de lampe de manière à réduire les changements de la concentration de ladite espèce de gaz à IR activé au fil du temps.

**2.** Méthode selon la revendication 1, dans laquelle ledit matériau catalytique (26) est opérationnel de manière à accroître la stabilité spectrale de ladite lampe en réduisant les changements du degré d'auto-absorption dans ledit mélange de gaz au fil du temps.

**3.** Méthode selon la revendication 1, dans laquelle ledit matériau catalytique (26) est enduit sur une paroi intérieure de ladite enveloppe (12).

**4.** Méthode selon la revendication 1, dans laquelle le matériau catalytique (26) est choisi dans un groupe comprenant l'or, l'argent, le rhodium, l'iridium, le palladium, le platine et le nickel.

**5.** Méthode selon la revendication 1 et dans laquelle l'étape consistant à inclure un matériau catalytique (26) au sein de ladite enveloppe de lampe (12) permet au volume de ladite lampe d'être réduit en comparaison avec une lampe construite sans inclure un tel matériau catalytique.

**6.** Méthode selon la revendication 5, dans laquelle ledit volume de lampe est inférieur à environ 6 millilitres.

**7.** Méthode selon la revendication 1 et dans laquelle l'étape consistant à inclure un matériau catalytique (26) au sein de ladite enveloppe de lampe permet à la concentration de gaz à IR activé d'être réduite en comparaison avec une lampe construite sans inclure un tel matériau catalytique (26).

**8.** Méthode selon l'une quelconque des revendications précédentes, dans laquelle ladite espèce de gaz à IR activé est le dioxyde de carbone.

**9.** Méthode selon la revendication 8, dans laquelle la concentration dudit dioxyde de carbone est inférieure à environ 5 %.

**10.** Lampe à décharge de gaz excitée électriquement, comprenant :

une enveloppe de lampe (12) contenant un mélange de gaz (16) comprenant ladite ou lesdites espèces de gaz à IR activé, ladite espèce de gaz étant telle que ladite lampe fournit une caractéristique de sortie d'émission spontanée à un état de base lorsqu'elle est électriquement excitée ; et
des électrodes (18, 20) externes à ladite enveloppe (12) pour l'excitation de ladite ou desdites espèces de gaz à IR activé ;
ladite lampe étant **caractérisée par** un matériau catalytique (26) situé au sein de ladite enveloppe de lampe (12) de manière à réduire les changements de la concentration de ladite espèce de gaz à IR activé au fil du temps.

**11.** Lampe selon la revendication 10, dans laquelle ledit catalyseur (26) est opérationnel de manière à accroître la stabilité spectrale de ladite lampe en réduisant les changements du degré d'auto-absorption dans ledit mélange de gaz au fil du temps.

**12.** Lampe selon la revendication 10, dans laquelle ledit matériau catalytique (26) est enduit sur une paroi intérieure de ladite enveloppe (12).

**13.** Lampe selon la revendication 10, dans laquelle le matériau catalytique (26) est choisi dans un groupe comprenant l'or, l'argent, le rhodium, l'iridium, le palladium, le platine et le nickel.

**14.** Lampe selon l'une quelconque des revendications 10 à 13, dans laquelle ladite espèce de gaz à IR activé est le dioxyde de carbone.

**15.** Méthode selon la revendication 8 comme applicable à la revendication 7 et dans laquelle ladite concentration réduite de dioxyde de carbone réduit l'auto-absorption de ladite lampe de manière à ce qu'un analyseur de gaz utilisant le spectre d'émission de dioxyde de carbone de ladite lampe montre des profondeurs de courbe d'absorption accrues en comparaison avec un analyseur utilisant une lampe construite sans inclure un tel matériau catalytique (26).

# FIG. 1

EP 1 230 669 B1

**EP 1 230 669 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5300859 A
- US 4757512 A
- US 4756000 A, J. Macken
- GB 1591709 A, R.S. Webley
- EP 0415600 A, R.C. Sharp
- US 4490482 A, P. Mathieu
- US 4639432 A, A. Holt et al.
- US 5300839 A